(19) Europäisches Patentamt
European Patent Office
Office européen des brevets

(11) Publication number: **0 174 121 B1**

(12) # EUROPEAN PATENT SPECIFICATION

(45) Date of publication of patent specification: **05.02.92**

(51) Int. Cl.⁵: **C01B 33/34**, C01B 35/12, C01B 33/20, C01G 17/00, C01G 37/14, C01G 49/00, B01J 29/28, B01J 29/04, C07C 2/86, C07C 6/12, C07C 2/00

(21) Application number: **85305825.3**

(22) Date of filing: **16.08.85**

(54) **A zeolite, a method of its synthesis, and organic conversion therewith.**

(30) Priority: **21.08.84 US 642930**
**21.08.84 US 642961**
**21.08.84 US 642962**
**21.08.84 US 642963**
**21.08.84 US 642964**
**21.08.84 US 642965**

(43) Date of publication of application:
**12.03.86 Bulletin 86/11**

(45) Publication of the grant of the patent:
**05.02.92 Bulletin 92/06**

(84) Designated Contracting States:
**BE DE FR GB IT NL SE**

(56) References cited:
**EP-A- 0 049 386**
**US-A- 4 016 245**
**US-A- 4 146 584**

(73) Proprietor: **MOBIL OIL CORPORATION**
**150 East 42nd Street**
**New York New York 10017(US)**

(72) Inventor: **Valyocsik, Ernest William**
**960 Randolph Drive**
**Yardley Pennsylvania 19067(US)**
Inventor: **Page, Nancy Marie**
**289 Flint Court**
**Yardley Pennsylvania 19067(US)**

(74) Representative: **Cooper, John Anthony et al**
**Mobil Court 3 Clements Inn**
**London WC2A 2EB(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 174 121 B1

## Description

This invention relates to a novel zeolite, to a method for its synthesis and to its use in catalytic conversion of organic compounds.

Zeolitic materials, both natural and synthetic, have been demonstrated in the past to have catalytic properties for various types of hydrocarbon conversion. Certain zeolitic materials are ordered, porous crystalline aluminosilicates having a definite crystalline structure as determined by X-ray diffraction, within which there are a large number of smaller cavities which may be interconnected by a number of still smaller channels or pores. These cavities and pores are uniform in size within a specific zeolitic material. Since the dimensions of these pores are such as to accept for adsorption molecules of certain dimensions while rejecting those of larger dimensions, these materials have come to be known as "molecular sieves" and are utilized in a variety of ways to take advantage of these properties.

Such molecular sieves, both natural and synthetic, include a wide variety of positive ion-containing crystalline aluminosilicates. These aluminosilicates can be described as a rigid three-dimensional framework of $SiO_4$ and $AlO_4$ in which the tetrahedra are cross-linked by the sharing of oxygen atoms whereby the ratio of the total aluminum and silicon atoms to oxygen atoms is 1:2. The electrovalence of the tetrahedra containing aluminum is balanced by the inclusion in the crystal of a cation, for example an alkali metal or an alkaline earth metal cation. This can be expressed wherein the ratio of aluminum to the number of various cations, such as Ca/2, Sr/2, Na, K or Li, is equal to unity. One type of cation may be exchanged either entirely or partially with another type of cation utilizing ion exchange techniques in a conventional manner. By means of such cation exchange, it has been possible to vary the properties of a given aluminosilicate by suitable selection of the cation. The spaces between the tetrahedra are occupied by molecules of water prior to dehydration.

Prior art techniques have resulted in the formation of a great variety of synthetic zeolites. The zeolites have come to be designated by letter or other convenient symbols, as illustrated by zeolite A (U. S. Patent 2,882,243), zeolite X (U. S. Patent 2,882,244), zeolite Y (U. S. Patent 3,130,007), zeolite ZK-5 (U. S. Patent 3,247,195), zeolite ZK-4 (U. S. Patent 3,314,752), zeolite ZSM-5 (U. S. Patent 3,702,886), zeolite ZSM-11 (U. S. Patent 3,709,979), zeolite ZSM-12 (U. S. Patent 3,832,449), zeolite ZSM-20 (U. S. Patent 3,972,983), ZSM-35 (U. S. Patent 4,016,245), ZSM-38 (U. S. Patent 4,046,859), and zeolite ZSM-23 (U. S. Patent 4,076,842).

The $SiO_2/Al_2O_3$ mole ratio of a given zeolite is often variable. For example, zeolite X can be synthesized with $SiO_2/Al_2O_3$ ratios of from 2 to 3; zeolite Y, from 3 to about 6. In some zeolites, the upper limit of the $SiO_2/Al_2O_3$ ratio is unbounded. ZSM-5 is one such example wherein the $SiO_2/Al_2O_3$ ratio is at least 5 and up to infinity. U. S. Patent 3,941,871 (Re. 29,948) discloses a porous crystalline silicate made from a reaction mixture containing no deliberately added alumina and exhibiting the X-ray diffraction pattern characteristic of ZSM-5 type zeolites.U. S. Patents 4,061,724, 4,073,865 and 4,104,294 describe crystalline silicates or organosilicates of varying alumina and metal content.

A number of synthetic zeolites have been prepared which may be said to be isostructural with naturally occurring zeolites. Zeolites ZSM-35 and ZSM-38 are, for instance, ferrierite-type zeolites. Zeolite ZK-20 (U.S. Patent 3,459,676) is described as being isostructural with the naturally occurring zeolite levynite.

Although zeolites include materials containing silica and alumina, it is recognized that the silica and alumina portions may be replaced in whole or in part with other oxides. More particularly, $GeO_2$ is an art recognized substitute for $SiO_2$ and $B_2O_3$, $Cr_2O_3$, $Fe_2O_3$, and $Ga_2O_3$ are art recognized replacements for $Al_2O_3$. Accordingly, the term zeolite is used herein to connote not only materials containing silicon and, optionally, aluminum atoms in the crystalline lattice structure thereof, but also materials which contain suitable replacement atoms for such silicon and/or aluminum. On the other hand, the term aluminosilicate zeolite is used herein to define zeolite materials consisting essentially of silicon and, optionally, aluminum atoms in the crystalline lattice structure thereof, as opposed to materials which contain substantial amounts of suitable replacement atoms for such silicon and/or aluminum.

The present invention resides in a novel porous crystalline zeolite, hereinafter referred to as zeolite ZSM-57, having a molar ratio of $XO_2$: $Y_2O_3$ of at least 4, wherein X represents silicon and/or germanium and Y represents aluminum, boron, chromium, iron and/or gallium, said porous crystalline zeolite having at least the X-ray diffraction lines as set forth in Table 1 below but not having an X-ray diffraction line at an interplanar D-spacing of 6.61 ± 0.15 Ångstrom.

Preferably, the molar ratio of $XO_2$: $Y_2O_3$ is in the range of 8 to 200 and most preferably $XO_2$ is silica and $Y_2O_3$ is alumina.

As is well known in the art, the preferred method for identifying a zeolite is by means of its X-ray diffraction pattern. Zeolites in accordance with the present invention have the characteristic X-ray diffraction

lines set out in Table 1.

TABLE 1

| d ± delta d(Ångstrom) | Intensity Range |
|---|---|
| 11.36 ± 0.23 | M-VS |
| 9.41 ± 0.19 | M-VS |
| 7.12 ± 0.15 | M-S (shoulder) |
| 6.95 ± 0.14 | M-S |
| 5.74 ± 0.12 | M |
| 5.68 ± 0.12 | W-M (shoulder) |
| 5.42 ± 0.11 | M-S |
| 4.81 ± 0.10 | W-M |
| 3.98 ± 0.08 | VW-M |
| 3.84 ± 0.08 | M-S (shoulder) |
| 3.79 ± 0.08 | VS |
| 3.64 ± 0.08 | W |
| 3.55 ± 0.08 | S |
| 3.48 ± 0.08 | S-VS |
| 3.36 ± 0.07 | W |
| 3.14 ± 0.07 | M-S |
| 3.06 ± 0.07 | W |
| 2.949 ± 0.06 | VW |
| 2.316 ± 0.05 | VW |
| 1.935 ± 0.04 | W |

In particular, it is to be noted that ZSM-57 is distinguished from ferrierite-type zeolites, such as ZSM-35, by the absence of a line at 6.61 ± 0.15 Ångstrom (13.09 - 13.71 2 Theta). The set of diffraction lines for ZSM-57 may, therefore, be considered to be a subset of the set of diffraction lines for ZSM-35.

Moreover, it will be understood that not all zeolites of the ZSM-57 structure will generate exactly the same X-ray diffraction data. For example, variations can occur which are attributable to the presence of impurities, e.g., in the form of occluded materials or crystalline intergrowths. The sodium forms in comparison with other cationic forms of otherwise identical zeolites reveal substantially the same patterns with some minor shifts in interplanar spacing and variation in relatiave intensity. Other minor variations can occur, depending on the silicon to aluminum ratio of the particular sample, as well as its degree of thermal treatment.

The following Table 2 gives X-ray diffraction data for the uncalcined, as synthesized form of the zeolite prepared in accordance with Example 4, set forth hereafter.

TABLE 2

| Interplanar D-spacing(Å) | Degrees 2 Theta | Relative Intensity, I/Io |
|---|---|---|
| 11.37 | 7.77 | 19.3 |
| 9.41 | 9.39 | 29.6 |
| 7.11 | 12.44 | 16.2 |
| 6.97 | 12.69 | 25.2 |
| 5.74 | 15.43 | 28.4 |
| 5.69 | 15.56 | 16.5 |
| 5.42 | 16.32 | 38.2 |
| 4.70 | 18.84 | 9.5 |
| 3.83 | 23.22 | 39.5 |
| 3.78 | 23.49 | 100.0 |
| 3.74 | 23.75 | 12.9 |
| 3.64 | 24.45 | 13.1 |
| 3.56 | 25.00 | 48.1 |
| 3.48 | 25.60 | 56.2 |
| 3.36 | 26.53 | 7.9 |
| 3.13 | 28.46 | 34.2 |
| 3.04 | 29.36 | 10.1 |
| 2.95 | 30.30 | 5.1 |
| 2.81 | 31.77 | 1.0 |
| 2.66 | 33.61 | 2.8 |

The following Table 3 gives X-ray diffraction data for the calcined form of the zeolite prepared in accordance with Example 3, set forth hereinafter.

TABLE 3

| Interplanar D-spacing(Å) | Degrees 2 Theta | Relative Intensity, I/Io |
|---|---|---|
| 11.25 | 7.85 | 63.2 |
| 9.97 | 8.86 | 2.7 |
| 9.37 | 9.46 | 6.41 |
| 7.04 | 12.56 | 40.0 |
| 6.91 | 12.80 | 50.2 |
| 5.70 | 15.53 | 30.0 |
| 5.64 | 15.69 | 24.3 |
| 5.39 | 16.44 | 32.6 |
| 4.78 | 18.55 | 18.7 |
| 4.68 | 18.94 | 6.3 |
| 4.58 | 19.36 | 1.5 |
| 3.91 | 22.78 | 9.9 |
| 3.81 | 23.34 | 37 9 |
| 3.76 | 23.64 | 100.0 |
| 3.71 | 23.95 | 12.0 |
| 3.62 | 24.58 | 12.9 |
| 3.53 | 25.18 | 55.3 |
| 3.46 | 25.75 | 56.0 |
| 3.34 | 26.66 | 15.5 |
| 3.31 | 26.90 | 10.1 |
| 3.12 | 28.61 | 42.7 |
| 3.30 | 29.42 | 13.5 |
| 3.02 | 29.48 | 17.6 |
| 2.93 | 30.46 | 7.4 |
| 2.80 | 31.95 | 1.8 |
| 2.73 | 32.79 | 1.0 |

These values were determined by standard techniques. The radiation was the K-alpha doublet of copper and a diffractometer equipped with a scintillation counter and an associated computer was used. The peak heights, I, and the positions as a function of 2 theta, where theta is the Bragg angle, were determined using algorithms on the computer associated with the spectrometer. From these, the relative intensities, 100 $I/I_o$, where $I_o$ is the intensity of the strongest line or peak, and d (obs.) the interplanar spacing in Ångstrom units (Å), corresponding to the recorded lines, were determined. In Table 2, the relative intensities are given in terms of the strongest line being taken as 100.0.

When synthesized, ZSM-57 will contain organic directing agent bound to the anionic sites in the crystal lattice and, hence prior to use, the as synthesized zeolite is calcined to remove the organic material, preferably at 200-900 °C.

The original alkali metal cations of the as synthesized zeolite ZSM-57 can he replaced in accordance with techniques well known in the art, at least in part, by ion exchange with other cations. Preferred replacing cations include metal ions, hydrogen ions, hydrogen precursor, e.g. ammonium ions and mixtures thereof. Particularly preferred cations are those which render the zeolite catalytically active, especially for hydrocarbon conversion. Replacing cations include hydrogen, rare earth metals and metals of Groups IA, IIA, IIIA, IVA, IB, IIB, IIIB, IVB and VIII of the Periodic Table of the Elements.

A typical ion exchange technique would be to contact the synthetic zeolite with a salt of the desired replacing cation or cations. Examples of such salts include the halides, e.g. chlorides, nitrates and sulfates.

Zeolite ZSM-57 sorbs significant amounts of commonly used test adsorbate materials, i.e. cyclohexane, n-hexane and water. Sorption capacities for the zeolite of the present invention may range at room temperature as follows:

| Adsorbate | Capacity, Wt. Percent |
|---|---|
| n-hexane | 6 - 7 |
| cyclohexane | 3 - 6 |
| water | 5 - 8 |

wherein cyclohexane and n-hexane sorption are measured at 20 Torr (2.7 kPa) and water sorption is measured at 12 Torr (1.6 kPa).

The zeolite can also be used as a catalyst in intimate combination with a hydrogenating component such as tungsten, vanadium, molybdenum, rhenium, nickel, cobalt, chromium, manganese, or a noble metal such as platinum or palladium where a hydrogenation-dehydrogenation function is to be performed. Such component can be exchanged into the composition to the extent atom Y, e.g., aluminum, is in the structure, impregnated therein or intimately physically admixed therewith. Such component can also be impregnated in or on to it such as for example, by, in the case of platinum, treating the zeolite with a solution containing a platinum metal-containing ion. Thus, suitable platinum compounds include chloroplatinic acid, platinous chloride and various compounds containing the platinum amine complex.

The zeolite ZSM-57 when employed either as an adsorbent or as a catalyst in an organic compound conversion process should be dehydrated, at least partially. This can be done by heating to a temperature in the range of 200°C to 595°C in an inert atmosphere, such as air, nitrogen, etc. and at atmospheric, subatmospheric or superatmospheric pressures for between 30 minutes and 48 hours. Dehydration can also be performed at room temperature merely by placing the zeolite in a vacuum, but a longer time is required to obtain a sufficient amount of dehydration.

The zeolite ZSM-57 can be prepared from a reaction mixture containing sources of alkali metal ions (Z), an oxide of Y, an oxide of X, a divalent N,N,N,N',N',N'-hexaethylpentane-diammonium cation (R), and water. The reaction mixture may comprise an appropriate selection of reactants, capable of forming the zeolite and having a composition falling within the following ranges:

| Reactants | Broad | Preferred |
|---|---|---|
| $SiO_2/Al_2O_3$ | 20-200 | 40-100 |
| $H_2O/SiO_2$ | 10-200 | 20-50 |
| $OH^-/SiO_2$ | 0-3 | 0.1-0.5 |
| $Z/SiO_2$ | 0-3 | 0.1-2 |
| $R/SiO_2$ | 0.01-2 | 0.1-1 |

wherein R and Z are as above defined. The as-synthesized zeolites may have a composition, expressed in terms of moles of oxides on an anhydrous basis, as follows:

$$(0\text{-}15)RO:(0\text{-}5)Z_2O:100SiO_2:(0.5\text{-}25)Al_2O_3$$

where R and Z are as defined above.

The divalent N,N,N,N',N',N'-hexaethylpentane-diammonium (hereinafter referred to as Hexaethyl-Diquat-5) cation may be supplied by suitable compounds of the formula:

$$X(C_2H_5)_3N^+(CH_2)_5N^+(C_2H_5)_3X'$$

where X and X' are the same or different and are appropriate counterbalancing anions such as fluoride, chloride, bromide, iodide, hydroxide, acetate, sulfate or carboxylate. Most preferably, the dibromide is employed and is conveniently produced by reaction of 1.5-dibromopentane with triethylamine.

Crystallization of the zeolite ZSM-57 can be carried out at either static or stirred conditions in a suitable reactor vessel, such as for example, polypropylene jars or teflon (registered Trade Mark) lined or stainless steel autoclaves. A useful range of temperatures for crystallization is from 80°C to 350°C for a time of 12 hours to 200 days. Thereafter, the crystals are separated from the liquid and recovered. The composition can be prepared utilizing materials which supply the appropriate oxides. Such compositions may include sodium silicate, silica hydrosol, silica gel, silicic acid, sodium hydroxide, a source of aluminum, and an appropriate organic compound. It should be realized that the reaction mixture component oxides can be supplied from more than one source. The reaction mixture can be prepared either batchwise or continuously. Crystal size and crystallization time of the crystalline zeolite of the present invention will vary with the nature of the reaction mixture employed and the crystallization conditions.

In all cases, synthesis of the zeolite crystals is facilitated by the presence of at least 0.01 wt. percent, preferably 0.10 wt. percent and still more preferably 1 wt. percent, seed crystals (based on total weight) of crystalline product.

The zeolite crystals can be shaped into a wide variety of particle forms. Generally speaking, the particles can be in the form of a powder, a granule, or a molded product. In cases where the catalyst is molded, such as by extrusion, the crystals can be extruded before drying or partially dried and then extruded.

In the case of many catalysts, it is desired to incorporate the zeolite with another material resistant to the temperatures and other conditions employed in organic conversion processes. Such materials include active and inactive material and synthetic or naturally occurring zeolites as well as inorganic materials such as clays, silica and/or metal oxides, e.g. alumina. The latter may be either naturally occurring or in the form of gelatinous precipitates or gels including mixtures of silica and metal oxides. Use of an active material in conjunction with the zeolite crystal, i.e. combined therewith, tends to improve the conversion and/or selectivity of the catalyst in certain organic conversion processes. Inactive materials suitably serve as diluents to control the amount of conversion in a given process so that products can be obtained economically and orderly without employing other means for controlling the rate of reaction. These materials may be incorporated into naturally occurring clays, e.g. bentonite and kaolin, to improve the crush strength of the catalyst under commercial operating conditions. Said materials, i.e. clays, oxides, etc., function as binders for the catalyst. It is desirable to provide a catalyst having good crush strength because in commercial use it is desirable to prevent the catalyst from breaking down into powder-like materials. These clay binders have been employed normally only for the purpose of improving the crush strength of the catalyst.

Naturally occurring clays which can be composited with the present zeolite include the montmorillonite and kaolin families which include the subbentonites, and the kaolins commonly known as Dixie, McNamee, Georgia and Florida clays or others in which the main mineral constituent is halloysite, kaolinite, dickite, nacrite, or anauxite. Such clays can be used in the raw state as originally mined or initially subjected to calcination, acid treatment or chemical modification. Binders useful for compositing with the present zeolite also include inorganic oxides, notably alumina.

Porous matrix materials which can be composited with the present zeolite include silica-alumina, silica-magnesia, silica-zirconia, silica-thoria, silica-beryllia, silica-titania as well as ternary compositions such as silica-alumina-thoria, silica-alumina-zirconia, silica-alumina-magnesia and silica-magnesia-zirconia. The relative proportions of finely divided crystalline material and inorganic oxide gel matrix vary widely, with the zeolite content ranging from 1 to 90 percent by weight and more usually, particularly when the composite is prepared in the form of beads, in the range of 2 to 80 weight percent of the composite.

The zeolite ZSM-57 is useful as a catalyst component for a variety of organic, e.g. hydrocarbon, compound conversion processes. Such conversion processes include cracking hydrocarbons with reaction conditions including a temperature of from 300°C to 700°C, a pressure of from 10 to 3040 kPa (0.1 to 30 atmospheres) and a weight hourly space velocity of from 0.1 to 20; dehydrogenating hydrocarbon compounds with reaction conditions including a temperature of from 300°C to 700°C, a pressure of from 10 to 1013 kPa (0.1 to 10 atmospheres) and a weight hourly space velocity of from 0.1 to 20; converting paraffins to aromatics with reaction conditions including a temperature of from 100°C to 700°C, a pressure of from 10 to 6080 kPa (0.1 to 60 atmospheres), a weight hourly space velocity of from 0.5 to 400 and a hydrogen/hydrocarbon mole ratio of from 0 to 20; converting olefins to aromatics, e.g. benzene, toluene and xylenes, with reaction conditions including a temperature of from 100°C to 700°C, a pressure of from 10 to 6080 kPa (0.1 to 60 atmospheres), a weight hourly space velocity of from 0.5 to 400 and a hydrogen/hydrocarbon mole ratio of from 0 to 20; converting alcohols, e.g. methanol, or ethers, e.g. dimethylether, or mixtures thereof to hydrocarbons including aromatics with reaction conditions including a temperature of from 275°C to 600°C, a pressure of from 51 to 5066 kPa (0.5 to 50 atmospheres) and a liquid hourly space velocity of from 0.5 to 100; isomerizing xylene feedstock components with reaction conditions including a temperature of from 230°C to 510°C, a pressure of from 304 to 3546 kPa (3 to 35 atmospheres), a weight hourly space velocity of from 0.1 to 200 and a hydrogen/hydrocarbon mole ratio of from 0 to 100; disproportionating toluene with reaction conditions including a temperature of from 200°C to 760°C, a pressure of from 101 to 6080 kPa (1 to 60 atmospheres) and a weight hourly space velocity of from 0.08 to 20; alkylating aromatic hydrocarbons, e.g. benzene and alkylbenzenes, in the presence of an alkylating agent, e.g. olefins, formaldehyde, alkyl halides and alcohols, with reaction conditions including a temperature of from 340°C to 500°C, a pressure of from 101 to 20265 kPa (1 to 200 atmospheres), a

weight hourly space velocity of from 2 to 2000 and an aromatic hydrocarbon/alkylating agent mole ratio of from 1/1 to 20/1; and transalkylating aromatic hydrocarbons in the presence of polyalkylaromatic hydrocarbons with reaction conditions including a temperature of from 340 °C to 500 °C, a pressure of from 101 to 20265 kPa (1 to 200 atmospheres), a weight hourly space velocity of from 10 to 1000 and an aromatic hydrocarbon/polyalkylaromatic hydrocarbon mole ratio of from 1/1 to 16/1.

Particular catalytic conversions for which the zeolite ZSM-57 can be used include (i) toluene disproportionation, (ii) toluene alkylation with methanol, (iii) propane (e.g., essentially pure propane) conversion to hydrocarbon mixtures enriched in BTX and (iv) upgrading refinery off-gas to liquid products enriched in BTX. It will be understood that BTX stands for aromatic hydrocarbon mixtures composed of two or more of benzene, toluene, xylene and ethylbenzene. The refinery off-gas is composed primarily of $C_1$-$C_3$ hydrocarbons and, optionally, hydrogen. This refinery off-gas will comprise at least 10% by weight of olefins (i.e. ethylene and/or propylene).

In order to more fully illustrate the nature of the invention and the manner of practicing same, the following examples are presented. In the examples, whenever adsorption data are set forth for comparison of sorptive capacities for water, cyclohexane and/or n-hexane, they were determined as follows:

A weighed sample of the calcined adsorbant was contacted with the desired pure adsorbate vapor in an adsorption chamber, evacuated to 1 mm and contacted with 12 mm Hg of water vapor or 20 mm Hg of n-hexane, or cyclohexane vapor, pressures less than the vapor-liquid equilibrium pressure of the respective adsorbate at room temperature. The pressure was kept constant (within about ± 0.5 mm) by addition of adsorbate vapor controlled by a manostat during the adsorption period, which did not exceed about 8 hours. As adsorbate was adsorbed by the zeolite, the decrease in pressure caused the manostat to open a valve which admitted more adsorbate vapor to the chamber to restore the above control pressures. Sorption was complete when the pressure change was not sufficient to activate the manostat. The increase in weight was calculated as the adsorption capacity of the sample in g/100 g of calcined adsorbant.

When Alpha Value is examined, it is noted that the Alpha Value is an approximate indication of the catalytic cracking activity of the catalyst compared to a standard catalyst and it gives the relative rate constant (rate of normal hexane conversion per volume of catalyst per unit time). It is based on the activity of the highly active silica-alumina cracking catalyst taken as an Alpha Value of 1 (Rate Constant = 0.016 $sec^{-1}$). The Alpha Test is described in U.S. Patent 3,354,078 and in The Journal of Catalysis, Vol. IV, pp. 522-529 (August 1965). The Constraint Index is a measure of the selectivity of the particular catalyst and it involves conversion of normal hexane and 3-methylpentane. This test is described in many U.S. patents, including 4,231,899 and 4,288,647.

EXAMPLES 1-8

Synthesis reaction mixtures were prepared with compositions indicated in TABLE 4. The mixtures were prepared with Q-brand sodium silicate (PQ Corporation: 27.8% $SiO_2$, 8.4% $Na_2O$), $Al_2(SO_4)_3.16H_2O$, Hexaethyl-Diquat-5 (bromide salt) and water. The mixtures were maintained at 160 °C for a number of days in a stainless steel, stirred (400 rpm) autoclave when crystallization was complete. The solids were separated from any unreacted components by filtration and then water washed, followed by drying at 110 °C.

TABLE 4

| Synthesis of Zeolite Samples (160° C, stirred) | | | | | | |
|---|---|---|---|---|---|---|
| Mixture Composition (Mole Ratios)[a] | | | | | | |
| Example No. | $SiO_2$ | $H_2O$ | $OH^-$ | $Na^+$ | $R^b$ | Time, |
| | $Al_2O_3$ | $SiO_2$ | $SiO_2$ | $SiO_2$ | $SiO_2$ | Days |
| 1 | 90 | 40 | 0.40 | 0.59 | 0.10 | 5 |
| 2 | 90 | 40 | 0.30 | 0.59 | 0.10 | 6 |
| 3 | 60 | 40 | 0.30 | 0.59 | 0.10 | 7 |
| 4 | 60 | 40 | 0.30 | 0.59 | 0.08 | 5 |
| 5 | 60 | 40 | 0.20 | 0.59 | 0.10 | 6 |
| 6 | 60 | 40 | 0.30 | 0.59 | 0.10 | 4 |
| 7 | 50 | 40 | 0.30 | 0.59 | 0.10 | 4 |
| 8 | 40 | 40 | 0.30 | 0.59 | 0.15 | 5 |

[a] Q-brand sodium silicate; $Al_2(SO_4)_3 . 16H_2O$.
[b] $R = (C_2H_5)_3N^+(CH_2)_5N^+(C_2H_5)_3$ = Hexaethyl-Diquat-5 (bromide salt).

The zeolite ZSM-57 was produced in each of Examples 1-8, although Examples 1 and 2 also produced a detectable amount of alpha quartz, the amount of alpha quartz in the Example 1 zeolite being merely a trace.

The directing agent N,N,N,N',N',N'-hexaethylpentanediammonium dibromide employed in crystallizing the zeolite of the present invention was prepared by refluxing overnight 1,5-dibromopentane with excess triethylamine in absolute ethanol. Analytical data for zeolite samples are compiled in Table 5. $SiO_2/Al_2O_3$ ratios for the zeolite range from 41-69. Aluminum present in the zeolite appears to be present as framework aluminum.

Also in Table 5, the compositions of the samples have been calculated on the basis of 100-$(SiO_2 + AlO_2^-)$ tetrahedra. If it is assumed that the diquaternary cation employed to direct the crystallization is trapped intact within the structure during synthesis, then, from the analytical data, one can calculate an average of 2-3 Hexaethyl-Diquat-5 cations per 100 tetrahedra in the zeolite structure.

TABLE 5

| Analytical Data for Zeolite Samples | | | | | | | | |
|---|---|---|---|---|---|---|---|---|
| Sample | C | Moles per mole $Al_2O_3$ | | | Composition[a] | | | |
| | N | $N_2O$ | $Na_2O$ | $SiO_2$ | Al | $Na^+$ | N | $R^b$ |
| | | | | | 100 $T_d$ | 100 $T_d$ | 100 $T_d$ | 100 $T_d$ |
| 1 | 10.2 | 1.3 | 0.84 | 69 | 2.8 | 2.4 | 3.6 | 2.2 |
| 2 | 8.6 | 1.3 | 0.53 | 68 | 2.9 | 1.5 | 3.7 | 1.9 |
| 3 | 8.7 | 1.2 | 0.28 | 43 | 4.4 | 1.2 | 5.4 | 2.7 |
| 4 | 10.1 | 1.2 | 0.63 | 45 | 4.3 | 2.7 | 5.2 | 3.1 |
| 5 | 8.6 | 1.5 | 0.33 | 48 | 4.0 | 1.3 | 6.1 | 3.1 |
| 6 | 7.3 | 1.4 | 0.31 | 41 | 4.6 | 1.4 | 6.4 | 2.8 |
| 7 | 10.8 | 1.0 | 0.21 | 43 | 4.4 | 0.92 | 4.6 | 2.9 |
| 8 | 7.7 | 1.5 | 0.98 | 36 | 5.3 | 5.2 | 7.9 | 3.6 |

a. Calculated on the basis of $100(SiO_2 + AlO_2^-)$tetrahedra.
b. $R = (C_2H_5)_3N^+(CH_2)_5N^+(C_2H_5)_3$.

As indicated previously herein, Table 2 sets forth the X-ray powder diffraction pattern data for the uncalcined, as synthesized form of the zeolite prepared in accordance with Example 4. Table 3 sets forth the X-ray powder diffraction pattern data for the calcined zeolite prepared in accordance with Example 3.

This Example 3 sample was calcined in air for six hours at 550° C. Clearly, the framework structure of this zeolite is stable to high temperature air calcination.

From SEM photomicrographs the zeolite crystals produced under these conditions appear to have a platelet morphology.

Characterization data for the Example 3 zeolite are set forth in Table 6. The H-form of the zeolite sorbed 7.1 wt% n-hexane, 4.7 wt% cyclohexane, and 6.7 wt% water at 25° C. Catalytic data is also set forth in Table 6. More particularly, Table 6 shows that the Example 3 zeolite ($SiO_2/Al_2O_3$ = 43) in the H-form also showed molecular shape selectivity (Constraint Index = 6.3 at 343° C [650° F]) and high cracking activity ( alpha = 225).

TABLE 6. Characterization Data for Example 3 Zeolite

Sample No. Example 3
Form: Hydrogen; $SiO_2/Al_2O_3$ = 43

I. Sorptions

| | Wt.%; 25°C[a] |
|---|---|
| N-hexane | 7.1 |
| Cyclohexane | 4.7 |
| Water | 6.7 |

a. Hydrocarbons, 20 mm; water, 12 mm

II. Catalytic Data

Alpha = 225
Constraint Index = 6.3[b]
$E_a$ = 13.8 Kcal/mole

b. 343°C (650°F)

EXAMPLE 9

The zeolite preparation procedure of Examples 1-8 was repeated except that sodium aluminate was used in place of $Al_2(SO_4)_3 \cdot 16H_2O$ and silica sol (30% $SiO_2$) was used in place of Q-brand sodium silicate. As shown in the results set forth in Table 7, only about 10% by weight of the zeolite of the present invention was obtained after 3 days.

TABLE 7

| Synthesis of Zeolite of the Present Invention (160° C, stirred) | | | | | | | |
|---|---|---|---|---|---|---|---|
| Mixture Composition (Mole Ratios)[a] | | | | | | | |
| Example No. | $SiO_2$ / $Al_2O_3$ | $H_2O$ / $SiO_2$ | $OH^-$ / $SiO_2$ | $Na^+$ / $SiO_2$ | $R^b$ / $SiO_2$ | Time / Days | Product |
| 9 | 60 | 40 | 0.30 | 0.33 | 0.10 | 3 | 10% Crystn. |

a. Silica sol (30% $SiO_2$); $NaAlO_2$.
b. R = $(C_2H_5)_3N^+(CH_2)_5N^+(C_2H_5)_3$ = Hexaethyl-Diquat-5 (bromide salt).

EXAMPLE 10

Samples of the zeolite of Example 6 were tested for catalytic activity. These crystals were pelletized, sized to 14/25 mesh (US Sieve Series) and screened in a quartz microreactor for activity and selectivity in a variety of para selective alkylation reactions. From SEM pictures it was estimated that the crystal dimensions of this zeolite preparation had a shortest crystal dimension of about 0.1 microns. Therefore, for comparison, a ZSM-5 crystal which had a $SiO_2/Al_2O_3 = 40$ and a shortest crystal dimension of 0.1 microns was also evaluated. Comparison of these two catalysts for selective toluene disproportionation, toluene alkylation with ethylene and toluene alkylation with methanol are presented in Tables 8-10, respectively.

TABLE 8. Toluene Disproportionation[1]

| Catalyst | Example 6 Zeolite | | | HZSM-5 | | | |
|---|---|---|---|---|---|---|---|
| Temp. °C | 450 | 500 | 600 | 450 | 500 | 550 | 600 |
| Toluene Conv., wt% | 45.0 | 43.1 | 33.4 | 19.1 | 34.8 | 44.3 | 46.7 |
| Product Selectivity | | | | | | | |
| Benzene | 42.3 | 46.7 | 47.6 | 43.7 | 47.4 | 44.3 | 58.4 |
| Ethylbenzene | 0.3 | 0.3 | 0.1 | 0.2 | 0.3 | 0.5 | 0.2 |
| Xylene | 48.3 | 48.0 | 48.2 | 54.2 | 49.4 | 41.5 | 37.7 |
| $C_9$+ | 9.0 | 4.8 | 4.2 | 1.8 | 2.7 | 3.6 | 3.7 |
| Xylene Isomer Distribution | | | | | | | |
| para | 24.0 | 23.9 | 23.4 | 24.1 | 23.6 | 23.5 | 23.3 |
| meta | 52.5 | 52.0 | 51.8 | 52.7 | 52.9 | 52.3 | 51.7 |
| ortho | 23.6 | 24.1 | 24.8 | 23.2 | 23.5 | 24.2 | 24.9 |

[1] Toluene WHSV = 5.5 $hr^{-1}$

TABLE 9. Toluene Alkylation with Ethylene[1]

| Catalyst | Example 6 Zeolite | | ZSM-5 | |
|---|---|---|---|---|
| Temp. °C | 400 | 450 | 400 | 450 |
| Toluene Conv., wt% | 23.0 | 17.1 | 17.3 | 19.1 |
| Product Selectivity | | | | |
| Benzene | 20.9 | 18.9 | 20.0 | 32.9 |
| Ethylbenzene/Xylenes | 33.2 | 28.6 | 26.5 | 36.5 |
| Ethyltoluene | 36.0 | 42.6 | 40.9 | 19.4 |
| $C_9$+ | 7.0 | 8.0 | 7.2 | 7.7 |
| Ethyltoluene Isomer Distribution | | | | |
| para | 30.1 | 30.3 | 28.1 | 29.0 |
| meta | 53.4 | 52.9 | 56.9 | 51.8 |
| ortho | 16.5 | 16.8 | 15.1 | 19.1 |

[1] Toluene WHSV = 8.8 $hr^{-1}$, $C_2H_4$ WHSV = 0.5 hr. Toluene/$C_2H_4$ molar ratio = 5/1

TABLE 10. Toluene Alkylation with Methanol[1]

| Catalyst | Example 6 Zeolite | | | HZSM-5 | | |
|---|---|---|---|---|---|---|
| Temp. °C | 400 | 500 | 600 | 400 | 500 | 600 |
| Toluene Conv., wt% | 31.9 | 35.3 | 30.2 | 21.7 | 34.2 | 38.5 |
| Product Selectivity | | | | | | |
| Benzene | 19.7 | 19.4 | 19.3 | 13.8 | 24.5 | 36.8 |
| Ethylbenzene | 0.4 | 0.1 | 0.1 | 0.9 | 0.5 | 0.3 |
| Xylene | 68.1 | 70.9 | 72.4 | 65.1 | 65.3 | 56.3 |
| $C_9+$ | 11.3 | 9.5 | 8.3 | 17.9 | 9.0 | 6.3 |
| Xylene Isomer Distribution | | | | | | |
| para | 23.5 | 23.4 | 23.4 | 24.1 | 23.8 | 23.7 |
| meta | 54.0 | 53.0 | 52.0 | 53.0 | 52.3 | 52.1 |
| ortho | 22.6 | 23.6 | 24.6 | 22.8 | 23.8 | 24.2 |

[1] Toluene/MeOH molar ratio = 4/1. Toluene WHSV = 15.8 $hr^{-1}$

EXAMPLE 11

To compare the shape-selective characteristics of the example 6 zeolite relative to ZSM-5, a solution of phenyldodecanes in benzene was prepared. This solution was then passed over the two catalysts, the Example 6 zeolite and ZSM-5. Isomer distributions of the starting material and of the phenyldodecanes after reaction over the 0.1 micron ZSM-5 crystal and the Example 6 crystal are summarized in Table 11. As these results show, both ZSM-5 and the Example 6 zeolite preferentially crack the smaller 2-phenyldodecane isomer. However, ZSM-5 appears to be more shape-selective than the Example 6 zeolite in this reaction as it completely removes all the 2-isomer. While the Example 6 zeolite does significantly reduce the amount of 2-isomer present relative to the other isomers, it does not completely remove all of the 2-isomer.

TABLE 11. Phenyldodecane Cracking

| Catalyst | FEED | Example 6 Zeolite | | ZSM-5 | |
|---|---|---|---|---|---|
| Temp., °C | | 150 | 200 | 150 | 200 |
| Isomer Distribution | | | | | |
| 6- | 15.9 | 27.4 | 27.1 | 26.3 | 26.5 |
| 5- | 14.6 | 26.4 | 26.1 | 26.5 | 27.4 |
| 4- | 14.6 | 23.1 | 22.9 | 25.3 | 24.8 |
| 3- | 16.5 | 18.2 | 17.1 | 22.0 | 21.3 |
| 2- | 37.4 | 4.8 | 6.7 | 0 | 0 |

As an additional comparison of the catalytic performance of the Example 6 zeolite and ZSM-5, the reaction

of propane over these two catalysts was studied. Results are summarized in Table 12. These results show that ZSM-57 has appreciable aromatization activity, but it does not appear to be as effective as ZSM-5 in aromatization reactions.

TABLE 12. Propane Cracking[1] over Example 6 Zeolite and ZSM-5

| Catalyst | Example 6 Zeolite | | ZSM-5 | |
|---|---|---|---|---|
| Temp., °C | 500 | 600 | 500 | 600 |
| Propane Conv. | 28.0 | 46.4 | 35.0 | 94.7 |
| Product Selectivity | | | | |
| $H_2$ | 1.6 | 3.1 | 1.7 | 3.6 |
| $C_1$ | 23.9 | 23.9 | 26.3 | 44.0 |
| $C_2$ | 16.9 | 5.2 | 21.8 | 14.4 |
| $C_2^=$ | 6.6 | 22.5 | 3.3 | 4.6 |
| $C_3^=$ | 8.2 | 16.0 | 3.5 | 1.6 |
| $C_4^\circ$ | 1.6 | 1.9 | 4.4 | 0 |
| $C_4^=$ | 11.5 | 0.7 | 11.0 | 0.2 |
| $C_5$ | 1.5 | 0.9 | 0.8 | 0 |
| $C_6$ | 0.7 | 0.7 | 0.3 | 0.2 |
| BZ | 3.0 | 6.5 | 5.6 | 17.8 |
| Tol | 5.6 | 6.5 | 10.4 | 9.4 |
| $C_8A$ | 5.1 | 4.5 | 8.4 | 2.6 |
| $C_9A$ | 2.7 | 2.0 | 1.7 | 0.4 |
| $C_{10}+$ | 11.2 | 5.7 | 0.9 | 1.2 |
| BTX Selectivity | 13.7 | 17.5 | 24.4 | 29.8 |
| $C_2^=$-$C_4^=$ Selectivity | 26.3 | 39.2 | 17.8 | 6.3 |

[1] Propane WHSV = 1.3 $hr^{-1}$

EXAMPLE 12

In addition, the catalytic performance of Example 6 zeolite was compared with that of a sample of ZSM-35, which is known to have a ferrierite-type structure. Results of these catalytic studies are summarized in Tables 13-15. For comparison purposes, catalytic data for ZSM-5 under the same conditions are also reported.

TABLE 13. Propane Cracking

| Catalyst | HZSM-35 | Example 6 Zeolite | HZSM-5 |
|---|---|---|---|
| Temp., °C | 600 | 600 | 600 |
| $C_3H_8$ WHSV | 1.3 | 1.3 | 1.3 |
| $C_3H_8$ Conv. | 58.3 | 69.3 | 99.3 |
| **Product Distribution** | | | |
| $H_2$ | 3.3 | 3.4 | 3.0 |
| $C_1$ | 38.8 | 28.1 | 41.8 |
| $C_2$ | 7.8 | 8.4 | 14.4 |
| $C_2$= | 28.7 | 12.9 | 3.6 |
| $C_3$= | 16.4 | 9.2 | 0.5 |
| $C_4$ | 0 | 11.7 | 0.05 |
| $C_4$= | 3.1 | 0.6 | 0.02 |
| $C_5$+ Alip. | 0.4 | 0.6 | 0.05 |
| Aroms. | 1.2 | 25.2 | 36.7 |

TABLE 14. Toluene Disproportionation

| Catalyst | ZSM-35 | | Example 6 Zeolite | | ZSM-5 | |
|---|---|---|---|---|---|---|
| Temp., °C | 500 | 600 | 500 | 600 | 500 | 600 |
| Toluene Conv., wt% | 2.7 | 2.7 | 43.1 | 33.4 | 34.8 | 46.7 |
| **Product Selectivity** | | | | | | |
| BZ | 53.5 | 66.7 | 46.7 | 47.6 | 47.4 | 58.4 |
| EB | 0.7 | 2.1 | 0.3 | 0.1 | 0.3 | 0.2 |
| Xyl | 45.8 | 29.7 | 48.0 | 48.2 | 49.4 | 37.7 |
| $C_9^+$ | 0 | 1.7 | 4.8 | 4.2 | 2.7 | 3.7 |
| **Isomer Distribution** | | | | | | |
| para | 39.1 | 44.4 | 23.9 | 23.4 | 23.6 | 23.3 |
| meta | 43.7 | 39.9 | 52.0 | 51.8 | 52.9 | 51.7 |
| ortho | 17.3 | 19.7 | 24.1 | 24.8 | 23.5 | 24.9 |

Toluene WHSV = 5.5 $hr^{-1}$

TABLE 15. Toluene Alkylation with Ethylene

| Catalyst | ZSM-35 | | Example 6 Zeolite | | ZSM-5 | |
|---|---|---|---|---|---|---|
| Temp., °C | 400 | 450 | 400 | 450 | 400 | 450 |
| Toluene Conv., wt% | 1.2 | 1.1 | 23.0 | 17.1 | 17.3 | 19.1 |
| Product Selectivity, wt% | | | | | | |
| BZ | 22.1 | 20.9 | 20.9 | 18.9 | 20.0 | 32.9 |
| EB/Xyl | 33.0 | 32.3 | 33.2 | 28.6 | 26.5 | 36.5 |
| ET | 44.9 | 46.8 | 36.0 | 42.6 | 40.9 | 19.4 |
| $C_9$+ | 0 | 0 | 7.0 | 8.0 | 7.2 | 7.7 |
| Isomer Distribution | | | | | | |
| para | 51.7 | 42.4 | 30.1 | 30.3 | 28.1 | 29.0 |
| meta | 37.8 | 44.1 | 53.4 | 52.9 | 56.9 | 51.8 |
| ortho | 10.6 | 13.5 | 16.5 | 16.8 | 15.1 | 19.1 |

Toluene WHSV = 8.8 $hr^{-1}$; $C_2H_4$ = 0.5 $hr^{-1}$. Toluene/$C_2H_4$ molar ratio = 5/1

EXAMPLE 13

Toluene Disproportionation

2.0 gm of the Example 6 catalyst (sized to 14/25 mesh [US Sieve Series]) was centered in a quartz microreactor with low surface area quartz chips being used to position the catalyst and fill void spaces. After calcination with air at 500°C for one hour, the reactor was flushed with nitrogen for approximately five minutes. The temperature was maintained at 500°C and toluene passed over the catalyst at the rate of 5.1 ml/hr (Toluene WHSV = 2.2 $hr^{-1}$). A liquid sample was collected for the last 5 min. of a 30 min. run and the composition of the liquid was determined by gas chromatography using a SCOT Bentone column. The temperature was then increased rapidly and succesively to 550° and 600°C. In a similar manner, liquid samples were taken for analysis during the last 5 min. of a 30 min. run. Results of these tests conducted are provided in Table 16.

TABLE 16. Toluene Disproportionation Over the Example 6 Zeolite

| | | | |
|---|---|---|---|
| Temperature, °C | 500° | 550° | 600° |
| Toluene Conversion, wt% | 53.2 | 54.2 | 53.9 |
| **Product Selectivities, wt%** | | | |
| Benzene | 49.1 | 51.3 | 53.2 |
| Ethylbenzene/Xylenes | 43.1 | 40.8 | 37.5 |
| $C_9$+ aromatics | 7.7 | 7.9 | 9.3 |
| **Isomer Distribution, wt%** | | | |
| p-xylene | 23.9 | 23.6 | 23.6 |
| m-xylene | 53.1 | 52.7 | 51.9 |
| o-xylene | 23.0 | 23.7 | 24.4 |

Toluene WHSV = 2.2 $hr^{-1}$

EXAMPLE 14

Toluene Alkylation with Methanol

A similar procedure to Example 12 was followed except, after calcination in air at 500°C for one hour, the temperature of the microreactor was adjusted to 400°C and a mixture of toluene and methanol (4:1 molar ratio toluene:methanol) is passed over the catalyst. After 25 min. on stream, a liquid sample was collected for 5 min. for analysis. The temperature was then successively increased to 500° and 600°C and the same procedure repeated. The results of the tests are summarized in Table 17.

TABLE 17. Toluene Alkylation With Methanol over Example 6 Zeolite

| | | | |
|---|---|---|---|
| Temperature, °C | 400° | 500° | 600° |
| Toluene WHSV, $hr^{-1}$ | 15.8 | 15.8 | 15.8 |
| Toluene Conversion, wt% | 31.9 | 35.3 | 30.2 |
| **Product Selectivities, wt%** | | | |
| Benzene | 19.7 | 19.4 | 19.3 |
| Xylenes | 68.2 | 70.9 | 72.4 |
| $C_9$+ Aromatics | 11.3 | 9.5 | 8.3 |
| **Isomer Distribution, wt%** | | | |
| p-xylene | 23.5 | 23.4 | 23.4 |
| m-xylene | 54.0 | 53.0 | 52.0 |
| o-xylene | 22.6 | 23.6 | 24.6 |

EXAMPLE 15

Propane Conversion

Again the procedure of Example 12 was followed, but propane was passed over the catalyst at 500° C. Samples of reactor effluent were taken after 30 min. on stream and after 2.0 hrs. on stream. The same procedure was repeated at 550° C and 600° C with calcination between each temperature change. Results of the tests are shown in Table 18.

TABLE 18. Propane Conversion Over Example 6 Zeolite

| Temp., °C | 500° | 500° | 550° | 550° | 600° | 600° |
|---|---|---|---|---|---|---|
| $C_3H_8$ WHSV, $hr^{-1}$ | 1.3 | 1.3 | 2.2 | 2.2 | 1.3 | 1.3 |
| Time on stream, hrs | 0.5 | 2.0 | 0.5 | 2.0 | 0.5 | 2.0 |
| Product Selectivity, wt% | | | | | | |
| $H_2$ | 1.7 | 1.6 | 1.9 | 2.0 | 3.9 | 3.1 |
| $CH_4$ | 26.8 | 23.9 | 33.7 | 30.1 | 32.3 | 23.8 |
| $C_2H_6$ | 21.5 | 16.9 | 19.0 | 14.1 | 9.6 | 5.2 |
| $C_2H_4$ | 4.6 | 6.6 | 7.6 | 11.2 | 14.8 | 22.5 |
| $C_3H_6$ | 4.5 | 8.2 | 7.8 | 12.7 | 10.6 | 16.0 |
| $C_4H_{10}$ | 1.1 | 1.6 | 1.9 | 2.7 | 1.2 | 1.9 |
| $C_4H_8$ | 10.6 | 11.5 | 6.5 | 5.8 | 0.7 | 0.7 |
| $C_5$ | 1.1 | 1.5 | 0.9 | 1.2 | 0.2 | 0.9 |
| $C_6$ | 0.4 | 0.8 | 0.9 | 1.2 | 0.4 | 0.7 |
| BZ | 4.0 | 3.0 | 4.5 | 3.8 | 7.8 | 6.5 |
| Tol | 7.6 | 5.6 | 7.3 | 6.8 | 9.0 | 6.5 |
| $C_8A$ | 7.4 | 5.1 | 5.5 | 5.8 | 5.3 | 4.6 |
| $C_9A$ | 2.6 | 2.7 | 1.7 | 1.9 | 1.6 | 2.0 |
| $C_{10}+$ | 6.2 | 11.2 | 0.8 | 0.9 | 2.7 | 5.7 |
| $C_3H_8$ Conversion | 36.5 | 28.0 | 44.0 | 32.3 | 67.0 | 46.4 |
| BTX Selectivity | 18.9 | 13.7 | 17.3 | 16.3 | 22.1 | 17.6 |
| $C_2^{=}-C_4^{=}$ Selec. | 19.6 | 26.3 | 22.0 | 29.6 | 26.0 | 39.1 |

EXAMPLE 16

Upgrading of Refinery Off Gas to Liquid Product Rich in BTX

Again the procedure of Example 12 was followed, but a synthetic refinery off gas was passed over the catalyst at 500° C. Reactor effluent was sampled at 0.5 hr. and 2.0 hrs. on stream. The catalyst was then calcined and the same procedure repeated at 600° C. Product distribution as well as the composition of the simulated refinery off gas are summarized in Table 19.

TABLE 19. Refinery Off Gas Upgrading Over Example 6 Zeolite

| | Feed | | | | |
|---|---|---|---|---|---|
| Temp., °C | | 500° | 500° | 600° | 600° |
| WHSV, $hr^{-1}$ | | 1.0 | 1.0 | 1.0 | 1.0 |
| Time on stream, hrs | | 0.5 | 2.0 | 0.5 | 2.0 |
| Composition, wt% | | | | | |
| $H_2$ | 1.6 | 2.2 | 2.2 | 2.8 | 2.8 |
| $CH_4$ | 38.0 | 42.5 | 41.6 | 45.2 | 42.5 |
| $C_2H_6$ | 22.0 | 24.2 | 23.6 | 24.0 | 23.0 |
| $C_2H_4$ | 15.3 | 1.2 | 1.6 | 4.4 | 7.0 |
| $C_3H_8$ | 6.6 | 14.7 | 12.7 | 3.2 | 5.8 |
| $C_3H_6$ | 16.6 | 1.0 | 2.1 | 1.7 | 4.5 |
| $C_4H_{10}$ | | 0.4 | 0.6 | 0.3 | 0.7 |
| $C_4H_8$ | | 2.6 | 2.9 | 0.2 | 0.3 |
| $C_5$ | | 0.4 | 0.7 | 0.1 | 0.1 |
| $C_6$ | | 0.2 | 0.4 | 0.04 | 0.3 |
| BZ | | 2.2 | 1.8 | 5.3 | 3.9 |
| Tol | | 4.0 | 3.9 | 5.0 | 4.2 |
| $C_8A$ | | 3.2 | 3.8 | 2.6 | 2.5 |
| $C_9A$ | | 0.8 | 1.2 | 0.7 | 0.8 |
| $C_{10}+$ | | 0.3 | 1.0 | 4.6 | 1.6 |
| gms BTX/100 gms feed | | 9.2 | 9.4 | 14.2 | 11.7 |

## Claims

**1.** A synthetic porous crystalline zeolite having a molar ratio of $XO_2$: $Y_2O_3$ of at least 4 wherein X represents silicon and/or germanium and Y represents a aluminum, boron, chromium, iron and/or gallium, said porous crystalline zeolite having at least the X-ray diffraction as set forth in Table 1 below, said zeolite not having an X-ray diffraction line at an interplanar D-spacing of 6.61 ± 0.15 Ångstrom:

TABLE 1

| d ± delta d(Ångstrom) | Intensity Range |
|---|---|
| 11.36 ± 0.23 | M-VS |
| 9.41 ± 0.19 | M-VS |
| 7.12 ± 0.15 | M-S (shoulder) |
| 6.95 ± 0.14 | M-2 |
| 5.75 ± 0.12 | M |
| 5.68 ± 0.12 | W-M (shoulder) |
| 5.42 ± 0.11 | M-S |
| 4.81 ± 0.10 | W-M |
| 3.98 ± 0.08 | VW-M |
| 3.84 ± 0.08 | M-S (shoulder) |
| 3.79 ± 0.08 | VS |
| 3.64 ± 0.08 | W |
| 3.55 ± 0.08 | S |
| 3.48 ± 0.08 | S-VS |
| 3.36 ± 0.07 | W |
| 3.14 ± 0.07 | M-S |
| 3.06 ± 0.07 | W |
| 2.949 ± 0.06 | VW |
| 2.316 ± 0.05 | VW |
| 1.935 ± 0.04 | W |

**2.** A crystalline zeolite according to claim 1, which is an aluminosilicate zeolite having a silica to alumina molar ratio of at least 4.

**3.** A crystalline zeolite according to claim 2 having a silica to alumina molar ratio of from 8 to 200.

**4.** A crystalline zeolite according to claim 1 and having the composition, expressed in terms of moles of oxides on an anhydrous basis, as follows:

(0-15)RO: (0-5)$Z_2O$ : 100$SiO_2$ : (0.5-25)$Al_2O_3$

where R is a cation of the formula

$(C_2H_5)_3N^+(CH_2)_5N^+(C_2H_5)_3$,

and Z is an alkali metal ion.

**5.** A method for preparing a synthetic porous crystalline zeolite according to claim 2, which comprises preparing a mixture containing sources of alkali metal ions, an oxide of aluminum, an oxide of silicon, water and an N,N,N,$N_1$,$N_1$,$N_1$-hexaethylpentanediammonium cation of the formula

$(C_2H_5)_3\ N^+(CH_2)_5\ N^+(C_2H_5)_3$

and maintaining said mixture under conditions to form said crystalline zeolite.

**6.** A method according to claim 5, wherein said mixture has a composition, in terms of moles, falling within the following ranges:
$SiO_2/Al_2O_3$ = 20 - 200
$H_2O/SiO_2$ = 10 - 200
$OH^-/SiO_2$ = 0 - 3
$Z/SiO_2$ = 0 - 3
$R/SiO_2$ = 0.01 - 2
where R is the organic cation and Z is the alkali metal ion.

7. A process for effecting catalytic conversion of an organic charge which comprises contacting said charge under catalytic conversion conditions with a catalyst comprising a synthetic porous crystalline zeolite according to any one of claims 1 to 3.

8. A process according to claim 7, wherein toluene is alkylated with methanol to produce xylenes, said process comprising contacting said toluene and said methanol with said catalyst under conditions comprising a temperature of from 340 °C to 500 °C, a pressure of from 101 to 20265 kPa (1 to 200 atmospheres), a weight hourly space velocity of from 2 to 2000 and a toluene to methanol mole ratio of from 1:1 to 20:1.

9. A process according to claim 7, wherein toluene is disproportionated to produce benzene and xylenes, said process comprising contacting said toluene with said catalyst under conditions comprising a temperature of from 200 °C to 760 °C, a pressure of from 101 to 6080 kPa (1 to 60 atmospheres) and a weight hourly space velocity of from 0.08 to 20.

10. A process according to claim 7, wherein propane is converted to produce a mixture comprising light olefins and aromatics, said process comprising contacting said propane with said catalyst under conditions comprising a temperature of from 100 °C to 700 °C, a pressure of from 10 to 6080 kPa (0.1 to 60 atmospheres), a weight hourly space velocity of from 0.5 to 400 and a hydrogen/propane mole ratio of from 0 to 20.

11. A process according to claim 7, wherein a refinery off gas is upgraded to produce liquid product enriched in benzene, toluene and xylenes, said process comprising contacting said off gas with said catalyst under conditions comprising a temperature of from 100 °C to 700 °C, a pressure of from 10 to 6080 kPa (0.1 to 60 atmospheres), a weight hourly space velocity of from 0.5 to 400 and a hydrogen to hydrocarbon mole ratio of from 0 to 20.

**Revendications**

1. Une zéolite cristalline poreuse synthétique ayant un rapport molaire $XO_2/Y_2O_3$ d'au moins 4, où X représente le silicium et/ou le germanium et Y représente un atome d'aluminium, de bore, de chrome, de fer et/ou de gallium, ladite zéolite cristalline poreuse ayant au moins l'image de diffraction aux rayons X telle que ressortant du tableau 1 ci-dessous, et ladite zéolite ne comportant pas de raie de diffraction aux rayons X correspondant à une distance D entre plans réticulaires de 6,71 ± 0,15 Angströms:

TABLEAU 1

| d ± Δd (Angströms) | Intervalle d'intensité |
|---|---|
| 11,36 ± 0,23 | M-VS |
| 9,41 ± 0,19 | M-VS |
| 7,12 ± 0,15 | M-S (épaulement) |
| 6,95 ± 0,14 | M-S |
| 5,74 ± 0,12 | M |
| 5,68 ± 0,12 | W-M (épaulement) |
| 5,42 ± 0,11 | M-S |
| 4,81 ± 0,10 | W-M |
| 3,98 ± 0,08 | VW-M |
| 3,84 ± 0,08 | M-S (épaulement) |
| 3,79 ± 0,08 | VS |
| 3,64 ± 0,08 | W |
| 3,55 ± 0 08 | S |
| 3,48 ± 0,08 | S-VS |
| 3,36 ± 0,07 | W |
| 3,14 ± 0,07 | M-S |
| 3,06 ± 0,07 | W |
| 2,949 ± 0,06 | VW |
| 2,316 ± 0,05 | VW |
| 1,935 ± 0,04 | W |

**2.** Une zéolite cristalline selon la revendication 1, consistant en une zéolite du type aluminosilicate ayant un rapport molaire silice/alumine d'au moins 4.

**3.** Une zéolite cristalline selon la revendication 2, ayant un rapport molaire silice/alumine de 8 à 200.

**4.** Une zéolite cristalline selon la revendication 1, et ayant la composition suivante, exprimée par le nombre de moles d'oxydes sur un base anhydre:

$(0\text{-}15)RO / (0\text{-}5)Z_2O / 100SiO_2 / (0,5\text{-}25)Al_2O_3$

où R est un cation de formule

$(C_2H_5)_3N^+(CH_2)_5N^+(C_2H_5)_3$

et Z est un ion de métal alcalin.

**5.** Un procédé de préparation d'une zéolite cristalline poreuse synthétique selon la revendication 2, qui consiste à préparer un mélange contenant des sources d'ions de métaux alcalins, un oxyde d'aluminium, un oxyde de silicium, de l'eau et un cation N,N,N,$N_1$,$N_1$,$N_1$-hexaéthylpentane-diammonium de formule:

$(C_2H_5)_3N^+(CH_2)_5N^+(C_2H_5)_3$

et à maintenir ledit mélange dans des conditions conduisant à la formation de ladite zéolite cristalline.

**6.** Un procédé selon la revendication 5, dans lequel ledit mélange a, en moles, une composition tombant dans les intervalles suivants:
$SiO_2/Al_2O_3$ 20 - 200
$H_2O/SiO_2$ 10 - 200
$OH^-/SiO_2$ 0 - 3
$Z/SiO_2$ 0 - 3
$R/SiO_2$ 0,01 - 2

où R est le cation organique et Z est l'ion de métal alcalin.

7. Un procédé pour mettre en oeuvre une conversion catalytique d'une charge organique, qui consiste à mettre en contact ladite charge, dans les conditions d'une conversion catalytique, avec un catalyseur comprenant une zéolite cristalline poreuse synthétique selon l'une quelconque des revendications 1 à 3.

8. Un procédé selon la revendication 7, dans lequel du toluène est alkylé avec du méthanol pour produire des xylènes, ledit procédé consistant à mettre en contact ledit toluène et ledit méthanol avec ledit catalyseur dans des conditions qui comprennent une température de 340 à 500 °C, une pression de 101 à 20265 kPa (1 à 200 atmosphères), une vitesse spatiale horaire pondérale de 2 à 2000 $h^{-1}$ et un rapport molaire toluène/méthanol de 1/1 à 20/1.

9. Un procédé selon la revendication 7, dans lequel du toluène est dismuté pour donner du benzène et des xylènes, ledit procédé consistant à mettre en contact ledit toluène avec ledit catalyseur dans des conditions qui comprennent une température de 200 à 760 °C, une pression de 101 à 6080 kPa (1 à 60 atmosphères) et une vitesse spatiale horaire pondérale de 0,08 à 20 $h^{-1}$.

10. Un procédé selon la revendication 7, dans lequel du propane est converti pour donner un mélange comprenant des oléfines légères et des aromatiques, ledit procédé consistant à mettre en contact ledit propane avec ledit catalyseur dans des conditions qui comprennent une température de 100 à 700 °C, une pression de 10 à 6080 kPa (0,1 à 60 atmosphères), une vitesse spatiale horaire pondérale de 0,5 à 400 $h^{-1}$ et un rapport molaire hydrogène/propane de 0 à 20.

11. Un procédé selon la revendication 7, dans lequel un gaz résiduel de raffinerie est valorisé pour donner un produit liquide enrichi en benzène, toluène et xylènes, ledit procédé consistant à mettre en contact ledit gaz résiduel avec ledit catalyseur dans des conditions qui comprennent une température de 100 à 700 °C, une pression de 10 à 6080 kPa (0,1 à 60 atmosphères), une vitesse spatiale horaire pondérale de 0,5 à 400 $h^{-1}$ et un rapport molaire hydrogène/hydrocarbures de 0 à 20.

## Patentansprüche

1. Synthetischer, poröser, kristalliner Zeolith mit einem Molverhältnis von $XO_2$ : $Y_2O_3$ von mindestens 4, worin X Silicium und/oder Germanium und Y Aluminium, Bor, Chrom, Eisen und/oder Gallium darstellen, wobei dieser poröse, kristalline Zeolith zumindest das nachfolgend in Tabelle 1 gezeigte Röntgenbeugungsdiagramm aufweist, wobei dieser Zeolith beim D-Netzebenenabstand von 6,61 ± 0,15 Angström keine Röntgenbeugungslinie aufweist:

Tabelle 1

| d± Delta d(Angström) | Intensitätsbereich |
|---|---|
| 11,36 ± 0,23 | M-VS |
| 9,41 ± 0,19 | M-VS |
| 7,12 ± 0,15 | M-S (Absatz) |
| 6,95 ± 0,14 | M-2 |
| 5,75 ± 0,12 | M |
| 5,68 ± 0,12 | W-M (Absatz) |
| 5,42 ± 0,11 | M-S |
| 4,81 ± 0,10 | W-M |
| 3,98 ± 0,08 | VW-M |
| 3,84 ± 0,08 | M-S (Absatz) |
| 3,79 ± 0,08 | VS |
| 3,64 ± 0,08 | W |
| 3,55 ± 0,08 | S |
| 3,48 ± 0,08 | S-VS |
| 3,36 ± 0,07 | W |
| 3,14 ± 0,07 | M-S |
| 3,06 ± 0,07 | W |
| 2,949 ± 0,06 | VW |
| 2,316 ± 0,05 | VW |
| 1,935 ± 0,04 | W |

**2.** Kristalliner Zeolith nach Anspruch 1, der ein Aluminosilicat Zeolith mit einem Silicciumdioxid/Aluminiumoxid-Molverhältnis von mindestens 4 ist.

**3.** Kristalliner Zeolith nach Anspruch 2, der ein Siliciumdioxid/Aluminiumoxid-Molverhältnis von 8 bis 200 aufweist.

**4.** Kristalliner Zeolith nach Anspruch 1, der die folgende Zusammensetzung aufweist, die auf die Mole der Oxide auf wasserfreier Basis bezogen ist:

$(0\text{-}15)RO : (0\text{-}5)Z_2O : 100SiO_2 : (0{,}5\text{-}25)Al_2O_3$

worin R ein Kation der Formel:

$(C_2H_5)_3N^+(CH_2)_5N^+(C_2H_5)_3$ und

Z ein Alkalimetallion sind.

**5.** Verfahren zur Herstellung eines synthetischen, porösen, kristallinen Zeoliths nach Anspruch 2, das die Herstellung einer Mischung, die Quellen von Alkalimetallionen, ein Oxid von Aluminium, ein Oxid von Silicium, Wasser und ein $N,N,N,N_1,N_1,N_1$,-Hexaethylpentandiammonium-Kation der Formel enthält:

$(C_2H_5)_3N^+(CH_2)_5N^+(C_2H_5)_3$

und das Halten dieser Mischung unter Bedingungen umfaßt, um den kristallinen Zeolith zu bilden.

**6.** Verfahren nach Anspruch 5, worin die Mischung eine auf die Mole bezogene Zusammensetzung aufweist, die innerhalb der folgenden Bereiche liegt:
$SiO_2/Al_2O_3$ = 20 - 200
$H_2O/SiO_2$ = 10 - 200
$OH^-/SiO_2$ = 0 - 3
$Z/SiO_2$ = 0 - 3
$R/SiO_2$ = 0,01 - 2

worin R das organische Kation und Z das Alkalimetallkation sind.

7. Verfahren zur Durchführung einer katalytischen Umwandlung einer organischen Beschickung, das den Kontakt dieser Beschickung bei katalytischen Umwandlungsbedingungen mit einem Katalysator umfaßt, der einen synthetischen, porösen, kristallinen Zeolith nach einem der Ansprüche 1 bis 3 umfaßt.

8. Verfahren nach Anspruch 7, worin Toluol mit Methanol alkyliert wird, um Xylole herzustellen, wobei dieses Verfahren den Kontakt von Toluol und Methanol mit dem Katalysator bei Bedingungen umfaßt, die eine Temperatur von 340 bis 500 °C, einen Druck von 101 bis 20265 kPa (1 bis 200 Atmoshphären), eine stündliche Gewichts-Raum-Geschwindigkeit von 2 bis 2000 und ein Toluol/Methanol-Molverhältnis von 1:1 bis 20:1 umfassen.

9. Verfahren nach Anspruch 7, worin Toluol disproportionert wird, um Benzol und Xylole zu erzeugen, wobei dieses verfahren den Kontakt von Toluol mit dem Katalysator bei Bedingungen umfaßt, die eine Temperatur von 200 bis 760 °C, einen Druck von 101 bis 6080 kPa (1 bis 60 Atmosphären) und eine stündliche Gewichts-Raum-Geschwindigkeit von 0,08 bis 20 umfassen.

10. Verfahren nach Anspruch 7, worin Propan umgewandelt wird, um eine Mischung herzustellen, die leichte Olefine und Aromaten umfaßt, wobei dieses Verfahren den Kontakt des Propans mit dem Katalysator bei Bedingungen umfaßt, die eine Temperatur von 100 bis 700 °C, einen Druck von 10 bis 6080 kPa (0,1 bis 60 Atmosphären), eine stündliche Gewichts-Raum-Geschwindigkeit von 0,5 bis 400 und ein Wasserstoff/Propan-Molverhältnis von 0 bis 20 umfassen.

11. Verfahren nach Anspruch 7, worin die Qualitätsverbesserung eines Raffinerieabgases erfolgt, um ein flüssiges Produkt zu erzeugen, das reich an Benzol, Toluol und Xylolen ist, wobei dieses Vefahren den Kontakt des Abgases mit einem Katalysator bei Bedingungen umfaßt, die eine Temperatur von 100 bis 700 °C, einen Druck von 10 bis 6080 kPa (0,1 bis 60 Atmosphären), eine stündliche Gewichts-Raum-Geschwindigkeit von 0,5 bis 400 und ein Wasserstoff/Kohlenwasserstoff-Molverhältnis von 0 bis 20 umfassen.